# EUROPÄISCHE PATENTSCHRIFT

(11) **EP 1 151 732 B1**
(45) Veröffentlichungstag und Bekanntmachung des Hinweises auf die Patenterteilung: **12.07.2006**
(21) Anmeldenummer: 01109193.1
(22) Anmeldetag: 12.04.2001
(51) Int. Cl.: A61F 2/30

(54) **Endoprothesenschaft und Verfahren zu seiner Herstellung**
Endoprosthetic shaft and method for making same
Tige endoprothétique et procede de fabrication de ladite tige

(30) Priorität: 04.05.2000 DE 10021697
(43) Veröffentlichungstag der Anmeldung: 07.11.2001
(73) Patentinhaber: Plus Orthopedics AG, 6343 Rotkreuz (CH)
(72) Erfinder: Semadeni, Marco, 8816 Hirzel (CH); Mayer, Jörg, 5702 Niederlenz (CH); Riner, Marc, 5018 Erlinsbach (CH); Berner, Werner, Dr., 5018 Erlinsbach (CH); Hänggli, Alfred, 5013 Niedergösgen (CH); Gruner, Heiko, Dr., 5712 Beinwil am See (CH)
(74) Vertreter: Popp, Eugen

(56) Entgegenhaltungen:
- EP-A- 0 197 441
- EP-A- 0 442 256
- EP-A- 0 642 774
- WO-A-93/19699
- WO-A-98/12994
- DE-A- 2 313 678
- FR-A- 2 509 986
- GB-A- 2 284 830

## Beschreibung

Die Erfindung betrifft einen Endoprothesenschaft nach dem Oberbegriff des Patentanspruches 1 sowie ein Verfahren zur Herstellung eines Endoprothesenschaftes nach dem Oberbegriff des Patentanspruches 18 bzw. des Patentanspruches 19.

Endoprothesen sind im Bereich der wiederherstellenden Chirurgie bzw. Orthopädie ein vielfach verwendetes Mittel, um durch Abnutzung oder sonstige schädigende Einflüsse nicht mehr im herkömmlichen Sinn einsetzbare Körperteile, insbesondere Gelenke, zu verstärken oder zu ersetzen.

Ein derartiger Gelenkersatz findet insbesondere in der Hüftchirurgie eine breite Anwendung, wobei hier insbesondere Hüftprothesen aus Metall oder Kunststoff zum Einsatz kommen.

Ein wesentlicher Aspekt bei der Verwendung einer künstlich hergestellten Prothese zur Implantation in ein lebendes Gewebe stellt die Bioverträglichkeit ihres Materials dar, wobei es hierbei insbesondere auf das außenliegende und somit mit dem Körpergewebe in Kontakt kommende Material ankommt.

Ein weiterer wesentlicher Aspekt bei der Verwendung einer Prothese ist deren Belastbarkeit, die auch unter einer Dauerbelastung über mehrere Jahrzehnte erhalten bleiben muß. Darüber hinaus sollte die äußere Beschaffenheit einer derartigen Prothese, insbesondere, ihre Abmessungen, ihre Elastizität und ihr Gewicht, im wesentlichen derjenigen ihres biologischen Pendants entsprechen.

Ursprünglich wurden Hüftendoprothesen aus Metall, die mit Kunststoff oder Keramik überzogen sein konnten, eingesetzt, da diese dauerhaft belastbar und formbeständig waren.

Derartige Prothesen mit Metallkern weisen jedoch Nachteile auf: So ist ihre Elastizität aufgrund ihres Metallkerns begrenzt, so daß bei einer Implantation einer metallkernbasierten Prothese in den Knochen, insbesondere Oberschenkelknochen, Zug- und Druckbelastungen richtungsabhängig nicht so aufgenommen werden können, wie dies ein Originalknochen täte, so daß insbesondere bei heftigen Bewegungen eine übermäßige Belastung des angrenzenden Knochens bis hin zu seiner Zerstörung zu befürchen ist. Zusätzlich können Metallimplantate auch Allergien auslösen.

Ein weiterer, wenngleich auch für den Träger weniger gravierender Nachteil von Metallprothesen ist, daß sie sich mit modernen Diagnoseverfahren, beispielsweise mit Computertomographie oder Kernspinresonanz nicht vernünftig respektive naturgetreu abbilden lassen und ihr Zustand beispielsweise gar nicht beurteilt werden kann.

Aufgrund dieser vielfältigen Nachteile von metallkernbasierten Prothesen wurden entsprechende Endoprothesensysteme auf einer im wesentlichen reinen Kunststoffbasis entwickelt.

So beschreibt die EP 442 256 A2 ein Knochenimplantat, das aus einem Grundkörper, insbesondere aus einem Kern und aus unidirektionalen Fasern besteht, wobei der Kern mit einem Fasergeflecht umgeben ist, das einen erhöhten Faseranteil aufweist, so daß an der Oberfläche des Fasergeflechts eine Strukturierung erreicht ist, die das Anwachsen des Knochenmaterials an das Implantat begünstigen soll.

Als nachteilig erweist sich bei diesem Knochenimplantat gemäß der EP 442 256 A2 der äußerst komplizierte Aufbau, was insbesondere dadurch zum Ausdruck kommt, daß das Flechtwerk mittels einer Flechtmaschine unter Einhaltung einer gleichmäßigen Spannung der Fasern auf den Grundkörper aufgebracht werden muß, der zur Einbringung der gewünschten Orientierung der Flechtfasern gleichzeitig geschwenkt werden muß. Darüber hinaus ist es notwendig, die Fasern an der Oberfläche des Implantats freizulegen, ohne diese hierbei zu beschädigen, um eine biokompatible Matrix zu erhalten, auf welche ein Knochen aufwachsen kann.

Die EP 197 441 A3 beschreibt ein Verfahren zur Behandlung von Knochenersatz-Implantaten, bei dem ein faserverstärkter Kunststoffkörper an seiner Oberfläche zumindest teilweise so behandelt wird, daß die Fasern zumindest teilweise freigelegt werden. Das Knochenersatzimplantat enthält hierbei einen Grundkörper aus Fasern, die unidirektional und annähernd parallel zur Schaftoberfläche angeordnet sind, oder aus Einzelfilamenten, wobei der Grundkörper mit einem mit Matrixmaterial getränkten Gewebe umspannt ist.

Auch dieses Implantat ist äußerst komplex aufgebaut und weist zudem eine unzureichende Biokompatibilität auf.

Demgemäß liegt der Erfindung die Aufgabe zugrunde, einen Endoprothesenschaft, der hinsichtlich seiner Stabilität und Belastbarkeit unter physiologischen Bedingungen und hinsichtlich seiner Biokompatibilität verbessert und darüber hinaus einfacher herstellbar ist, sowie ein Verfahren zu seiner Herstellung unter Vermeidung der vorgenannten Nachteile aufzuzeigen.

Diese Aufgabe wird durch einen Endoprothesenschaft gemäß Patentanspruch 1 sowie durch ein Verfahren gemäß Patentanspruch 18 bzw. Patentanspruch 19 gelöst.

Insbesondere wird die Aufgabe durch einen Endoprothesenschaft mit einem Grundkörper aus faserverstärktem Kunststoff gelöst, der mit Hilfe eines Spritzgießverfahrens als isoelastischer Körper ausgebildet ist. Dieser ist im Femur wirkenden physiologischen Kräfteverhältnissen angepaßt und erhält eine vorbestimmte Festigkeit im wesentlichen allein durch eine dicke Oberflächenschicht mit gleichförmig in Längsrichtung des Grundkörpers orientierten Verstärkungsfasern, insbesondere Kohlenstofffasern. Die radiale Erstreckung dieser Schicht ist über den größeren Teil der Länge des Grundkörpers größer als die radiale Erstreckung eines Kernbereichs mit nicht gleichförmig ausgerichteten Verstärkungsfasern.

In ihrem Verfahrensaspekt wird die Aufgabe durch ein Verfahren gelöst, bei dem eine Formgebung der Hüftprothese in einem Durchflußspritzgußzyklus durchgeführt wird, wobei eine eine Grundform der Hüftprothese im wesentlichen bildende Kunststoffmasse an einem Einlaßende in einer Negativform der Hüftprothese zugeführt und ein Überschußanteil der Kunststoffmasse an einem Auslaßende der Negativform der Hüftprothese abgeführt wird.

Alternativ wird die Aufgabe durch ein Gegentaktspritzgußverfahren gelöst, wobei eine den Grundkörper im wesentlichen bildende Kunststoffmasse wechselweise im Gegentakt von je zwei verschiedenen Enden einer Negativform der Hüftprothese zugeführt wird.

Ein wesentlicher Gedanke der Erfindung besteht darin, daß die Festigkeit des Endoprothesenschafts nicht wie bisher üblich durch einen harten Kern, insbesondere Metallkern, oder aber durch ein aufwendiges äußeres Verstärkungsfaser-Netzwerk bzw. -Geflecht, erreicht wird, sondern daß die Festigkeit im wesentlichen allein durch eine dicke Oberflächenschicht eines materialeinheitlichen Grundkörpers gewährleistet ist. Der wesentliche Aspekt hierbei ist, daß die Oberflächenschicht aus einem Kunststoff, in bevorzugter Weise aus Polyetheretherketon (PEEK) gebildet ist, der gleichförmig gerichtete Fasern, insbesondere Verstärkungsfasern enthält.

Gemäß einem weiteren wesentlichen Aspekt sind die Verstärkungsfasern in der dicken Oberflächenschicht in Längsrichtung des Grundkörpers ausgerichtet und im wesentlichen parallel zueinander angeordnet.

Die in dem Kernbereich des Endoprothesenschafts angeordneten Verstärkungsfasern folgen hierbei in einem an die dicke Oberflächenschicht angrenzenden Bereich im wesentlichen der Ausrichtung der in der Oberflächenschicht angeordneten Verstärkungsfasern, wobei sich bezüglich der Ausrichtung der Verstärkungsfasern ein im Längsschnitt parabelförmiges Profil ergibt.

Die Festigkeit des Endoprothesenschafts ist hierbei umso höher, je homogener das Gefüge gleichsinnig ausgerichteter Verstärkungsfasern ist und je größer das Verhältnis der dicken Oberflächenschicht mit gleichförmig ausgerichteten Verstärkungsfasern im Verhältnis zu der Dicke des Kernbereichs mit nicht gleichförmig ausgerichteten Verstärkungsfasern ist.

Als Fasermaterialien kommen hierbei Kohlenstoff-, Keramik-, Glas-, Aramid-Fasern jeweils allein oder auch in Kombination in Betracht. Diese Fasern können in Form von Langfasern und/oder Kurzfasern eingesetzt werden, wobei jedoch Fasern im Längenbereich von 10 µm bis 800 µm, bevorzugt im Bereich von 40 µm bis 700 µm und besonders bevorzugt im Bereich von 50 µm bis 560 µm, vorteilhaft sind.

Als Kunststoffe kommen neben dem bereits erwähnten Polyetheretherketon weiterhin Epoxidharze, Acrylharze, Polyolefine, Polyamide oder Polyimide jeweils allein oder in Kombination in Betracht, wobei sich jedoch der thermoplastische Kunststoff Polyetheretherketon (=Polyoxy-1,4-phenylen-oxy-1,4-phenylencarbonyl-1,4-phenylen) hinsichtlich seiner mechanischen, chemischen und thermischen Eigenschaften als bevorzugtes Material erwiesen hat. Als Fasermaterial zur Verstärkung des Kunststoffs sind Fasern auf Kohlenstoffbasis insbesondere aufgrund ihres geringen spezifischen Gewichts sowie ihrer ausgezeichneten Biokompatibilität, und des niedrigen Preises bevorzugt.

Gemäß einer bevorzugten Ausführungsform der Erfindung weist der Endoprothesenschaft eine dünne metallische und/oder keramische, im wesentlichen vollflächige Beschichtung des Grundkörpers auf seiner zum Kontakt mit dem Femur bestimmten Oberfläche zur Gewährleistung der Biokompatibilität auf. Der Vorteil dieser Beschichtung besteht darin, daß, je nach verwendetem Kunststoff mögliche Bioinkompatibilitäten vermieden werden, was insbesondere im Bereich der Kontaktfläche des Prothesenschaftes mit dem Femur entscheidend zur Einsetzbarkeit des Endoprothesenschaftes beiträgt.

Als Metalle kommen hierbei alle für diese Anwendung gängigen Metalle in Betracht, die unter physiologischen Bedingungen inert und/oder bioverträglich sind, wobei insbesondere Metalle oder Metalllegierungen auf Titanbasis geeignet sind.

Gemäß einer weiteren vorteilhaften Ausführungsform der Erfindung besteht die metallische Beschichtung aus einer inneren Hüllschicht und einer äußeren Strukturschicht. Der Vorteil dieser Ausführungsform liegt darin, daß bei einem derartigen zweischichtigen Aufbau der Metallschicht zunächst die innere Hüllschicht innig mit dem Kunststoff-Grundkörper verbunden ist, wobei beispielsweise Metallpartikel in die Oberfläche des faserverstärkten Kunststoffes eingeschmolzen sind.

Bei einem zweischichtigen Aufbau der Metallschicht ist es hierbei nicht zwingend nötig, daß die innere Hüllschicht vollflächig mit dem Grundkörper aus Kunststoff verbunden ist. So ist es beispielsweise möglich, daß die innere Hüllschicht lediglich netzartig oder in Form isolierter Verankerungspunkte als Basis für eine innige und festhaftende Anbringung der äußeren Strukturschicht dient.

Ein Vorteil dieser Ausführungsform ist es beispielsweise, daß auf diese Weise Bereiche, in denen die Endoprothese einer höheren Belastung ausgesetzt ist und in denen bei herkömmlichen Endoprothesen ein Abplatzen einer äußeren Schicht zu befürchten wäre, gezielt mit einer inneren Hüllschicht entsprechend verstärkt sein können.

Gemäß einer bevorzugten Ausführungsform der Erfindung weist die innere Hüllschicht hierbei eine Schichtdicke im Bereich von 40 µm bis zu 120 µm, bevorzugt im Bereich von 60 µm bis 100 µm und besonders bevorzugt im Bereich von 75 µm bis 85 µm, auf.

Des weiteren ist die Hüllschicht mit einer ersten vorbestimmten Rauhigkeit ausgebildet, die eine Rauhtiefe im Bereich von etwa 20 µm bis etwa 70 µm aufweist.

Aufgrund dieser geringen Schichtdicke der Hüllschicht, die hinsichtlich ihrer mechanischen Eigenschaften praktisch keinen Beitrag zu der Festigkeit des Endoprothesenschaftes liefert, ist es gewährleistet, daß die Elastizität des Endoprothesenschaftes durch diese Hüllschicht in keinster Weise beeinträchtigt wird. Durch die Rauhtiefe ist hingegen gewährleistet, daß die Strukturschicht, die auf der Hüllschicht angeordnet ist, gut mit dieser inneren Schicht verbunden ist.

Die Strukturschicht weist insbesondere eine Schichtdicke im Bereich von 150 µm bis 250 µm, bevorzugt im Bereich von 170 µm bis 230 µm und besonders bevorzugt im Bereich von 190 µm bis 210 µm auf und ist mit einer zweiten vorbestimmten Rauhigkeit im Bereich von 50 µm bis 100 µm Rauhtiefe ausgestattet.

Gemäß einer weiteren Ausführungsform der Erfindung ist auf mindestens einer metallischen Beschichtung eine keramische Beschichtung angeordnet, wobei die keramische Beschichtung ein Material auf Calciumphosphatbasis, insbesondere Hydroxylapatit aufweist.

Dieses Material erweist sich als besonders vorteilhaft, da es als ein auch natürlich im Knochen vorkommendes Material höchst biokompatibel ist.

Durch die Rauhtiefe, respektive die Rauhigkeit, welche die Strukturschicht aufweist, ist eine gute Haftung des keramischen Materials, das seinerseits eine Schichtdicke im Bereich von 40 bis 100 µm, bevorzugt im Bereich von 50 µm bis 80 µm und besonders bevorzugt im Bereich von 65 µm bis 75 µm aufweist, gewährleistet.

An dieser Stelle sei darauf hingewiesen, daß weder die Hüllschicht, noch die Strukturschicht, noch die keramische Beschichtung einen wesentlichen Beitrag zu den mechanischen Eigenschaften, insbesondere der Festigkeit des Endoprothesenschaftes liefern. Diese Festigkeit ist im wesentlichen auf der Struktur der dicken Oberflächenschicht und der darin gleichförmig in Längsrichtung des Grundkörpers orientierten Verstärkungsfasern basiert, wobei die Festigkeit beispielsweise durch eine Veränderung des Dickenverhältnisses der dicken Oberflächenschicht zu dem Kernbereich beinflußt werden kann, in welchem die Verstärkungsfasern nicht gleichförmig bezüglich der auf der Oberflächenschicht angeordneten Verstärkungsfasern angeordnet sind.

Der Endoprothesenschaft weist einen proximalen Bereich, einen distalen Bereich und einen zwischen dem distalen Bereich und dem proximalen Bereich liegenden Zwischenbereich auf, wobei gemäß einer weiteren Ausführungsform der Erfindung die innere Hüllschicht im wesentlichen nur in dem proximalen Bereich und dem Zwischenbereich angeordnet ist, während die Strukturschicht im wesentlichen nur in dem Zwischenbereich vorgesehen ist. Alternativ ist es auch möglich, die Strukturschicht ebenfalls in dem proximalen Bereich vorzusehen, wobei insbesondere bei einer nicht deckungsgleichen Anordnung von Hüllschicht und Strukturschicht die Hüllschicht vollflächig auf dem nicht von der Strukturschicht bedeckten proximalen Bereich ausgebildet ist.

Die keramische Schicht, speziell aus dem Material auf Kalziumphosphatbasis, ist gemäß einer weiteren erfindungsgemäßen Ausführungsform im wesentlichen nur in dem Zwischenbereich angeordnet - wobei auch eine Ausdehnung der keramischen Schicht auch auf den proximalen Bereich möglich ist.

Aufgrund dieser unterschiedlichen möglichen Anordnungen der Schichten und der damit verbundenen unterschiedlichen Oberflächeneigenschaften ist es möglich, die Einwachszone des Endoprothesenschaftes in den Knochen gezielt zu steuern und anzupassen, wobei aufgrund des isoelastischen Grundkörpers des Endoprothesenschafts die Biegeelastizität des Endoprothesenschafts nicht beeinträchtigt wird.

Der Verstärkungsfasergehalt des faserverstärkten Kunststoffs liegt im Bereich von 20% bis 80%, bevorzugt im Bereich von 30% bis 70% und besonders bevorzugt im Bereich von 40% bis 55%.

Ein Problem bei der Herstellung bisheriger Endoprothesen stellte die Erzielung der notwendigen Festigkeiten dar, die auch unter Dauerbelastung über einen sehr langen Zeitraum von bis zu mehreren Jahrzehnten erhalten bleiben sollten. Diese Festigkeiten sollten wie auch die üblichen mechanischen Eigenschaften weitgehend an die eines natürlichen lebenden Knochens angepaßt sein, so daß der Einsatz derartiger Endoprothesen nicht durch die mangelhafte mechanische Eigenschaften beschränkt ist. Wie bereits einleitend ausgeführt wurde, haben bisherige Endoprothesen zur Erreichung dieses Ziels einen relativ komplizierten Aufbau und sind dementsprechend aufwendig in der Fertigung. Insofern war es wünschenswert eine Endoprothesenschaft der oben genannten erfindungsgemäßen Art zu schaffen, der nicht nur optimierte mechanische und biokompatible Eigenschaften aufweist, wie dies erfindungsgemäß der Fall ist, sondern dies auch mit einem einfachen Verfahren zu bewerkstelligen.

Insofern ist ein weiterer erfindungswesentlicher Gedanke der Lösung der eingangs gestellten Aufgabe die Anwendung eines Verfahrens zur Herstellung eines metallkernfreien Endoprothesenschafts mit vorgenannten optimierten Eigenschaften, bei dem eine im wesentlichen parallele Faserorientierung, welche die notwendige Festigkeit des Endoprothesenschafts gewährleistet in einem Schritt erzeugt wird.

Wesentlich ist demgemäß, daß die Formgebung der Hüftprothese mittels eines Durchflußspritzgußverfahren durchgeführt wird, wobei eine eine Grundform der Hüftprothese im wesentlichen bildende Kunststoffmasse, welche als wesentliche Bestandteile sowie Etheretherkiton sowie Kohlenstofffasern aufweist, an einem Einlaßende in einer Negativform der Hüftprothese zugeführt wird, während ein Überschußanteil der Kunststoffmasse an einem Auslaßende der Negativform der Hüftprothese austritt.

Bei der Anwendung von konventionellem Spritzguß, bei welchem eine nur einseitig offene Negativform durch die einzige Öffnung mit einem faserverstärkten Kunststoff gefüllt wird, hat es sich als äußerst problematisch erwiesen, eine einheitliche homogene Faserausrichtung zu erreichen, die dem Produkt die für Endoprothesen notwendige Festigkeit verleiht. Insbesondere bei schwierigeren Geometrien ist eine gleichmäßige Auffüllung der Hohlform kaum möglich, und die in die Form gepresste Kunststoffmasse, inclusive deren Fasern, ist Scherkräften ausgesetzt ist, die zu Faserbrüchen bzw. zu Faserdesorientierungen führen. Dies ist insbesondere dadurch bedingt, daß die verpreßte Kunststoffmasse im Verlauf der Auffüllung das Hohlformende erreicht und eine Ablenkung beispielsweise in Richtung des Einlaßendes erfährt.

Ein wesentlicher Gedanke liegt nun darin, daß ein Durchflußspritzgußsystem geschaffen wird, bei dem eine Fasern enthaltende Kunststoffmasse an einem Einlaßende kontinuierlich in die Negativform zugeführt wird und diese gleichmäßig und gleichförmig ausfüllt und ein Anteil der Kunststoffmasse, der an das Ende der Negativform gelangt ist, ohne einen Gegenschub in Richtung Einlaßende zu erzeugen, aus der Negativform austreten kann. Die Kunststoffmasse wird bevorzugt bei einer Temperatur von im Bereich von 150° bis 450°C, speziell im Bereich von 400°C bis 335°C und besonders bevorzugt bei einer Temperatur von 415°C bis 430°C, verarbeitet und in die Negativform der Hüftprothese eingebracht, während die Negativform während des Füllvorgangs auf einer Temperatur im Bereich von 120°C, vorzugsweise im Bereich von 170°C bis 230°C und besonders bevorzugt im Bereich von 190°C bis 210°C, gehalten wird.

Auf diese Weise ist gewährleistet, daß eine Verfestigung der Kunststoffmasse in der Negativform von außen nach innen erfolgt, wobei der Kunststoff sich entlang der kälteren Wandung der Negativform abkühlt und erhärtet, und während des Erstarrungsprozesses der Kunststoffmasse durch noch flüssige bzw. plastische Kunststoffmasse, welche an der sich verfestigenden Kunststoffmasse vorbei fließt, eine Faserausrichtung stattfindet. Auf diese Weise ist gewährleistet, daß sich die in der Kunststoffmasse befindlichen Fasern einheitlich in Fließrichtung ausrichten und somit im wesentlichen parallel zueinander über das gesamte bisher verfüllte Volumen der Negativform im wesentlichen homogen und gleichförmig verteilt sind.

Die Geschwindigkeit des Verfestigungsprozesses kann hierbei über verschiedene Variable gesteuert werden, wobei insbesondere die Verarbeitungstemperatur der Kunststoffmasse und die Temperatur der Negativform variiert werden können.

Desweiteren ist es möglich, und ggf. zweckmäßig, die Ausflußgeschwindigkeit und/oder die Auslaßmenge der Kunststoffmasse aus dem Auslaßende der Negativform mittels zumindest einem Ventil zu regeln, wodurch einerseits ein Überschußanteil der Kunststoffmasse begrenzt werden als auch andererseits ein wohldefinierter vorbestimmter Gegendruck bezüglich der Fließrichtung in der Negativform der Hüftprothese aufgebaut werden kann. In einer vereinfachten Ausführung ist es auch möglich, anstelle eines Ventils einen Auslaß mit einem definierten Querschnitt vorzusehen.

Gemäß einem weiteren Aspekt der Erfindung wird die Kunststoffmasse während des Formbildungsvorganges mit konstanter Geschwindigkeit in die Negativform zugeführt.

Gemäß einer weiteren Ausführungsvariante der Erfindung ist es, möglich, den durch die Negativform fließenden Volumenstrom der Kunststoffmasse während des Formbildungsvorgangs konstant zu halten, wobei hierzu vor Beginn der Verfüllung die Zuführgeschwindigkeit an einer Injektionseinheit, vorzugsweise programmgesteuert, variabel gestaltet sein kann.

Erfindungsgemäß wird die Negativform vollständig mit der den Endprothesenschaft bildenden Kunststoffform aufgefüllt, wobei jedoch auch eine nur teilweise Verfüllung unter Erhalt eines Hohlkörpers möglich ist. So ist es auf diese Weise beispielsweise möglich bei wenig belasteten Endoprothesen das Gewicht der Prothese zu verringern, während deren Stabilität durch die in einer dicken Oberflächenschicht gleichförmigen Längsrichtung der Prothese ausgerichteten Verstärkungsfasern weitgehend erhalten bleibt.

Gemäß einem weiteren Aspekt des erfindungsgemäßen Verfahrens wird die oben erwähnte Hüllschicht in die Oberfläche des kohlenstofffaserverstärkten Kunststoffs eingeschmolzen, wodurch diese innigst mit dem Kunststoffkörper verbunden wird, so daß ein Abplatzen praktisch ausgeschlossen ist.

In einer vorteilhaften Variante wird die Hüllschicht hierbei in Form von Metallpartikeln auf die Oberflächenschicht des kunststofffaserverstärkten Kunststoffs aufgeschossen, so daß die Metallpartikel in den Kunststoff eindringen und eine innige Verbindung zwischen Kunststoff und Metall gewährleistet ist, so daß die Hüllschicht fest auf der Kunststoffschicht haftet. Gegebenenfalls ist der Vorgang des Aufschießens der Metallpartikel auf den Kunststoff unmittelbar von einem Einschmelzen der Metallpartikel in den Kunststoff gefolgt.

Ebenso ist es selbstverständlich möglich, die Metallpartikel auf den Kunststoff aufzupressen, bzw. den Kunststoff mit Metallpartikeln, die erhitzt sein können bespritzen.

Gemäß einem weiteren Aspekt der vorliegenden Erfindung wird ein Verfahren zur Herstellung eines metallkernfreien Endoprothesenschafts vorgeschlagen, bei welchem die Ausrichtung von Verstärkungsfasern in einer Kunststoffmatrix mittels eines Gegentaktspritzgußsystems erreicht wird, wobei eine eine Grundform der Hüftprothese im wesentlichen bildende Kunststoffmasse wechselweise im Gegentakt von je zwei verschiedenen Enden einer Negativform der Hüftprothese zugeführt wird.

Erfindungsgemäß wird hierbei die Kunststoffmasse zunächst auf einer Seite der Negativform durch ein Einlaßende in die Negativform der Hüftprothese eingebracht, während die Kunststoffmasse gleichzeitig auf einer gegenüberliegenden Seite der Negativform der Hüftprothese durch ein Auslaßende aus der Negativform abgezogen wird. Im Anschluß an diesen Vorgang wird Kunststoffmasse aus dem nun gefüllten zweiten Injektionssystem, welches zu Beginn des Verfüllvorgängs leer war, von der anderen Seite her in die Negativform der Hüftprothese eingespritzt, wobei wiederum auf der gegenüberliegenden Seite der Negativform Kunststoffmasse abgezogen wird. Auch bei dieser Verfahrensweise verfestigt sich der durch die Negativform fließende Kunststoff von außen nach innen, so daß der Durchflußkanal durch die Negativform immer enger wird, bis die Negativform der Hüftprothese vollständig aufgefüllt ist.

Auch hier ist es möglich, die Auffüllung der Negativform an einem zuvor definierten Punkt zu stoppen, so daß ein Hohlkörper entsteht.

Weitere Aspekte der Erfindung ergeben sich aus den Unteransprüchen sowie einem Ausführungsbeispiel, welches anhand der Abbildungen näher erläutert wird. Hierbei zeigen:
Figur 1 eine schematische Darstellung eines Endoprothesenschaftes gemäß einer Ausführungsform der Erfindung und
Figur 2 einen schematische Darstellung eines Endprothesenschaftes gemäß einer Ausführungsform der Erfindung, welche eine erfindungsgemäße Faserausrichtung von Verstärkungsfasern wiedergibt.

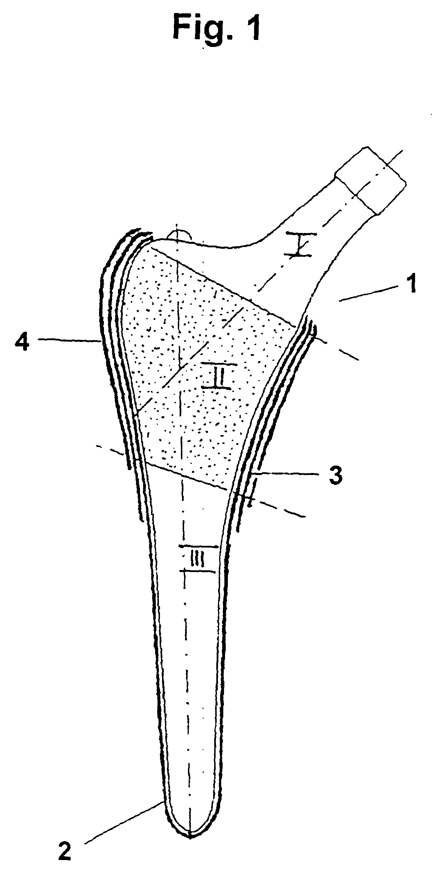

Figur 1 zeigt schematisch einen Hüftprothesenschaft, wobei ein distaler Bereich I, ein Zwischenbereich II und ein proximaler Bereich III markiert sind. Hierbei ist eine Hüllschicht 2 aus Titan vollflächig über den Zwischenbereich II und den proximalen Bereich III ausgebildet, während eine Titanstrukturschicht 3 sowie eine keramische Schicht 4 aus Hydroxylapatit im wesentlichen nur über dem Zwischenbereich angeordnet sind und sowohl in Richtung des proximalen Bereichs, als auch in Richtung des distalen Bereichs auslaufen.

Bei einem derart aufgebauten Hüftprothesenschaft, ist insbesondere durch die Hydroxlapatitschicht in dem Zwischenbereich gewährleistet, daß eine Knochanwachszone im wesentlichen auf den Zwischenbereich begrenzt ist, während der distale und insbesondere der proximale Bereich im Knochen geringfügig "beweglich" bleiben.

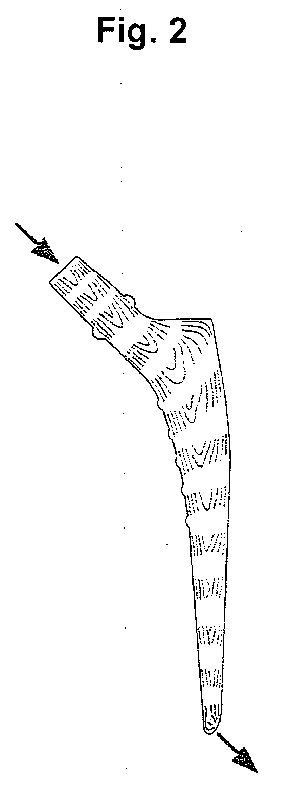

Figur 2 veranschaulicht schematisch die Faserausrichtung in einem erfindungsgemäßen Hüftprothesenschaft, wobei deutlich erkennbar ist, daß eine dicke Oberflächenschicht gleichförmig in Längsrichtung des Grundkörpers orientierte Verstärkungsfasern aufweist, und das lediglich in einem kleinen Kernbereich die Verstärkungsfaser nicht orientiert sind. Die Pfeile veranschaulichen die Durchfluß-, respektive Ausflußrichung des Kunststoffs durch die Negativform.

Zu der folgenden Tabelle werden desweiteren einige typische Verfahrensparameter für ein Durchflußspritzgußsystem wiedergegeben.

An dieser Stelle sei darauf hingewiesen, daß alle oben beschriebenen Teile für sich alleine gesehen und in jeder Kombination, insbesondere die in den Zeichnungen dargestellten Details als erfindungswesentlich beansprucht werden. Abänderungen hiervon sind dem Fachmann geläufig.

### Durchflußspritzgußsystem

| **Spritzgußbedingungen** | **Spritzgußeinheit** |
|---|---|
| Gefäßtemperatur (°C) | 370..430 |
| Temperatur der Schmelze (°C) | 370/390/410/430 |
| Temperatur der Form (°C) | 120/160/200/240 |
| Schneckengeschwindigkeit (Upm) | 100 |
| Zuführgeschwindigkeit (mm/s) | 40 |
| Zuführzeit (s) | 1.52 |
| Zuführdruck (bar) | 1190 |
| Durchflußgeschwindigkeit (mm/s) | 5/30/60/90/120 |
| Durchflußdruck (bar) | 1250/1400/1550/1700 |
| Ausflußvolumen (cm³) | 28/54/65/83 |
| Ausflußventil (mm²) | eingestellt |
| Haltedruck (bar) | 1450 |
| Haltezeit (s) | 40 |
| Zykluszeit (s) | 71..87 |

## Patentansprüche

1. Endoprothesenschaft, insbesondere Hüftprothesenschaft (1), mit einem Grundkörper aus faserverstärktem Kunststoff,
***dadurch gekennzeichnet, dass***
der Grundkörper durch ein Spritzgießverfahren als ein den physiologischen Kräfteverhältnissen im Femur angepasster isoelastischer Körper ausgebildet ist, der eine vorbestimmte Festigkeit im wesentlichen allein durch eine dicke Oberflächenschicht mit gleichförmig in Längsrichtung des Grundkörpers orientierten Verstärkungsfasern, insbesondere Kohlenstofffasern, erhält, wobei die radiale Erstreckung dieser Schicht über den größeren Teil der Länge des Grundkörpers größer als die radiale Erstreckung eines Kernbereichs mit nicht gleichförmig ausgerichteten Verstärkungsfasern ist.

2. Endoprothesenschaft nach Anspruch 1,
***gekennzeichnet durch***
eine dünne metallische und/oder keramische, im wesentlichen vollflächige Beschichtung des Grundkörpers auf seiner zum Kontakt mit dem Femur bestimmten Oberfläche zur Gewährleistung der Biokompatibilität.

3. Endoprothesenschaft nach Anspruch 2,
***dadurch gekennzeichnet, dass***
die metallische Beschichtung eine innere Hüllschicht (2) und eine äußere Strukturschicht (3) umfasst, wobei die Hüllschicht (2) und/oder die Strukturschicht (3) ein unter physiologischen Bedingungen inertes und/oder bioverträgliches Metall, insbesondere Titan oder eine Titanlegierung, enthält.

4. Endoprothesenschaft nach Anspruch 2 oder 3,
***dadurch gekennzeichnet, dass***
die keramische Beschichtung (4) ein Material auf Kalziumphosphatbasis, insbesondere Hydroxyldapatit, aufweist.

5. Endoprothesenschaft nach einem der Ansprüche 2 bis 4,
***dadurch gekennzeichnet, dass***
auf mindestens einer metallischen Beschichtung eine keramische Beschichtung (4) angeordnet ist.

6. Endoprothesenschaft nach einem der Ansprüche 2 bis 5,
***dadurch gekennzeichnet, dass***
die Hüllschicht (2) mit einer ersten vorbestimmten Rauhigkeit und die Strukturschicht (3) mit einer zweiten vorbestimmten Rauhigkeit ausgebildet ist.

7. Endprothesenschaft nach einem der Ansprüche 3 bis 6,
***dadurch gekennzeichnet, dass***
die Hüllschicht (2) mit einer Oberflächenschicht aus dem faserverstärkten Kunststoff innig verbunden, insbesondere aus in die Oberfläche des faserverstärkten Kunststoffs eingeschmolzenen Metallpartikeln, gebildet ist.

8. Endoprothesenschaft nach einem der Ansprüche 3 bis 7,
**dadurch *gekennzeichnet, dass***
die Hüllschicht (2) eine Schichtdicke im Bereich von 40 µm bis 120 µm, bevorzugt im Bereich von 60 µm bis 100 µm und besonders bevorzugt im Bereich von 75 µm bis 85 µm, aufweist.

9. Endoprothesenschaft nach einem der Ansprüche 3 bis 8,
***dadurch gekennzeichnet, dass***
die Hüllschicht (2) eine Rauhtiefe im Bereich von 20 µm bis 70 µm aufweist.

10. Endoprothesenschaft nach einem der Ansprüche 3 bis 9,
***dadurch gekennzeichnet, dass***
die Strukturschicht (3) eine Schichtdicke im Bereich von 150 µm bis 250 µm, bevorzugt im Bereich von 170 µm bis 230 µm und besonders bevorzugt im Bereich von 190 µm bis 210 µm, aufweist.

11. Endoprothesenschaft nach einem der Ansprüche 3 bis 10,
***dadurch* gekennzeichnet, *dass***
die Strukturschicht (3) eine Rauhtiefe im Bereich von 50 µm bis 180 µm aufweist.

12. Endoprothesenschaft nach einem der Ansprüche 2 bis 11,
***dadurch gekennzeichnet, dass***
die Schicht (4) aus dem Material auf Kalziumphosphatbasis eine Schichtdicke im Bereich von 40 µm bis 100 µm, bevorzugt im Bereich von 50 µm bis 80 µm und besonders bevorzugt im Bereich von 65 µm bis 75 µm, aufweist.

13. Endoprothesenschaft nach einem der Ansprüche 3 bis 12,
***dadurch gekennzeichnet, dass***
die Hüllschicht (2) im wesentlichen nur in einem proximalen Bereich und in einem zwischen einen distalen Bereich und dem proximalen Bereich liegenden Zwischenbereich vorgesehen ist.

14. Endoprothesenschaft nach einem der Ansprüche 3 bis 13,
***dadurch gekennzeichnet, dass***
die Strukturschicht (3) im wesentlichen nur in dem zwischen dem distalen Bereich und dem proximalen Bereich liegenden Zwischenbereich vorgesehen ist.

15. Endoprothesenschaft nach einem der Ansprüche 2 bis 14,
***dadurch gekennzeichnet, dass***
die keramische Beschichtung (4) im wesentlichen nur in dem zwischen dem distalen Bereich und dem proximalen Bereich liegenden Zwischenbereich vorgesehen ist.

16. Endoprothesenschaft nach einem der vorhergehenden Ansprüche,
***dadurch gekennzeichnet, dass***
ein Verstärkungsfasergehalt, insbesondere Kohlenstofffasergehalt des Kunststoffs im Bereich von 20 Gew.-% bis 80 Gew.-%, bevorzugt im Bereich von 30 Gew.-% bis 70 Gew.-% und besonders bevorzugt im Bereich von 40 Gew.-% bis 55 Gew.-%, liegt.

17. Endoprothesenschaft nach einem der vorhergehenden Ansprüche,
***dadurch gekennzeichnet, dass***
die Faserlänge der Verstärkungsfasern im Bereich von 10 µm bis 800 µm, bevorzugt im Bereich von 40 µm bis 700 µm und besonders bevorzugt im Bereich von 50 µm bis 560 µm, liegt.

18. Verfahren zur Herstellung eines Endoprothesenschafts, nach einem der vorangehenden Ansprüche,
***dadurch gekennzeichnet, dass***
eine Formgebung einer Hüftprothese (1) in einem Durchflussspritzgusszyklus durchgeführt wird, wobei eine eine Grundform der Hüftprothese (1) im wesentlichen bildende Kunststoffmasse an einem Einlassende in eine Negativform der Hüftprothese zugeführt und ein Überschussanteil der Kunststoffmasse an einem Auslassende der Negativform der Hüftprothese austritt.

19. Verfahren zur Herstellung eines Endoprothesenschafts, nach einem der Ansprüche 1 bis 17,
***dadurch gekennzeichnet, dass***
eine Formgebung des Grundkörpers in einem Gegentaktspritzgusszyklus durchgeführt wird, wobei eine eine Grundform der Hüftprothese (1) im wesentlichen bildende Kunststoffmasse wechselweise im Gegentakt von zwei verschiedenen Enden einer Negativform zugeführt wird.

20. Verfahren nach Anspruch 18,
***dadurch gekennzeichnet, dass***
die Kunstoffmasse kontinuierlich an einem Einlassende der Negativform zugeführt wird.

21. Verfahren nach Anspruch 18 oder 20,
***dadurch gekennzeichnet, dass***
die Kunststoffmasse während eines Formbildungsvorgangs mit konstanter Geschwindigkeit zugeführt wird.

22. Verfahren nach Anspruch 18 oder 20,
***dadurch gekennzeichnet, dass***
der durch die Negativform fließende Volumenstrom der Kunststoffmasse während des Formbildungsvorganges konstant gehalten wird.

23. Verfahren nach einem der Ansprüche 18 oder 20 bis 22,
***dadurch gekennzeichnet, dass***
eine Verfestigung der Kunststoffmasse in der Negativform von außen nach innen erfolgt, wobei die Zuführung der Kunststoffmasse solange erfolgt, bis eine vorbestimmte Verfüllung der Negativform erreicht ist.

24. Verfahren nach einem der Ansprüche 18 oder 20 bis 23,
***dadurch gekennzeichnet, dass***
die Negativform vollständig mit der Kunststoffmasse gefüllt wird.

25. Verfahren nach einem der Ansprüche 18 oder 20 bis 24,
**dadurch *gekennzeichnet, dass***
die Ausflussgeschwindigkeit und/oder die Auslassmenge der Kunststoffmasse aus dem Auslassende der Negativform mit zumindest einem Ventil geregelt wird.

26. Verfahren nach einem der Ansprüche 18 oder 20 bis 25,
***dadurch gekennzeichnet, dass***
die Kunststoffmasse bei einer Temperatur im Bereich von 350 °C bis 450 °C, bevorzugt im Bereich von 400 °C bis 335 °C und besonders bevorzugt bei einer Temperatur im Bereich von 415 °C bis 430 °C, in die Negativform der Hüftprothese eingebracht wird.

27. Verfahren nach einem der Ansprüche 18 oder 20 bis 26,
***dadurch gekennzeichnet, dass***
die Negativform während des Füllvorgang auf einer Temperatur im Bereich von 120 °C bis 250 °C, vorzugsweise im Bereich von 170 °C bis 230 °C und besonders bevorzugt im Beriech von 190 °C bis 210 °C, gehalten wird.

28. Verfahren nach einem der Ansprüche 18 bis 27,
***dadurch gekennzeichnet, dass***
die Hüllschicht (2) in die Oberfläche des kohlenstofffaserverstärkten Kunststoffs eingeschmolzen wird.

29. Verfahren nach einem der Ansprüche 18 bis 28,
***dadurch gekennzeichnet, dass***
die Hüllschicht (3) auf die Oberflächenschicht aus dem kohlenstofffaserverstärkten Kunststoff in Form von Metallpartikeln aufgeschossen und/oder aufgepresst und/oder aufgespritzt wird.

## Claims

1. Endoprosthesis stem, especially a hip prosthesis stem (1), having a main body of fibre-reinforced plastics material,
**characterized in that**
the main body, produced by an injection-moulding process, is in the form of an isoelastic body matched to the physiological force relationships in the femur, which body acquires a predetermined strength substantially solely as a result of a thick surface layer having reinforcing fibres, especially carbon fibres, oriented uniformly in the longitudinal direction of the main body, the radial extent of that layer over the greater part of the length of the main body being greater than the radial extent of a core region having reinforcing fibres that are not uniformly oriented.

2. Endoprosthesis stem according to claim 1,
**characterized by**
a thin metallic and/or ceramic coating which to ensure biocompatibility covers substantially the entire area of the main body on its surface intended for contact with the femur.

3. Endoprosthesis stem according to claim 2,
**characterized in that**
the metallic coating comprises an inner covering layer (2) and an outer structural layer (3), the covering layer (2) and/or the structural layer (3) containing a metal that is biocompatible and/or inert under physiological conditions, especially titanium or a titanium alloy.

4. Endoprosthesis stem according to claim 2 or 3,
**characterized in that**
the ceramic coating (4) comprises a material based on calcium phosphate, especially hydroxyl apatite.

5. Endoprosthesis stem according to any one of claims 2 to 4,
**characterized in that**
a ceramic coating (4) is arranged on at least one metallic coating.

6. Endoprosthesis stem according to any one of claims 2 to 5,
**characterized in that**
the covering layer (2) is provided with a first predetermined roughness and the structural layer (3) is provided with a second predetermined roughness.

7. Endoprosthesis stem according to any one of claims 3 to 6,
**characterized in that**
the covering layer (2) is intimately joined to a surface layer of the fibre-reinforced plastics material, and is especially formed from metal particles fused into the surface of the fibre-reinforced plastics material.

8. Endoprosthesis stem according to any one of claims 3 to 7,
**characterized in that**
the covering layer (2) has a layer thickness in the range of from 40 µm to 120 µm, preferably in the range of from 60 µm to 100 µm, especially in the range of from 75 µm to 85 µm.

9. Endoprosthesis stem according to any one of claims 3 to 8,
**characterized in that**
the covering layer (2) has a depth of roughness in the range of from 20 µm to 70 µm.

10. Endoprosthesis stem according to any one of claims 3 to 9,
**characterized in that**
the structural layer (3) has a layer thickness in the range of from 150 µm to 250 µm, preferably in the range of from 170 µm to 230 µm, especially in the range of from 190 µm to 210 µm.

11. Endoprosthesis stem according to any one of claims 3 to 10,
**characterized in that**
the structural layer (3) has a depth of roughness in the range of from 50 µm to 180 µm.

12. Endoprosthesis stem according to any one of claims 2 to 11,
**characterized in that**
the layer (4) of the calcium-phosphate-based material has a layer thickness in the range of from 40 µm to 100 µm, preferably in the range of from 50 µm to 80 µm, especially in the range of from 65 µm to 75 µm.

13. Endoprosthesis stem according to any one of claims 3 to 12,
**characterized in that**
the covering layer (2) is provided substantially only in a proximal region and in an intermediate region located between a distal region and the proximal region.

14. Endoprosthesis stem according to any one of claims 3 to 13,
**characterized in that**
the structural layer (3) is provided substantially only in the intermediate region located between the distal region and the proximal region.

15. Endoprosthesis stem according to any one of claims 2 to 14,
**characterized in that**
the ceramic coating (4) is provided substantially only in the intermediate region located between the distal region and the proximal region.

16. Endoprosthesis stem according to any one of the preceding claims,
**characterized in that**
the content of reinforcing fibres, especially carbon fibres, in the plastics material is in the range of from 20 % by weight to 80 % by weight, preferably in the range of from 30 % by weight to 70 % by weight, especially in the range of from 40 % by weight to 55 % by weight.

17. Endoprosthesis stem according to any one of the preceding claims,
**characterized in that**
the fibre length of the reinforcing fibres is in the range of from 10 µm to 800 µm, preferably in the range of from 40 µm to 700 µm, especially in the range of from 50 µm to 560 µm.

18. Process for the production of an endoprosthesis stem, in accordance with any one of the preceding claims,
**characterized in that**
moulding of a hip prosthesis (1) is carried out in a flow-through injection moulding cycle, wherein a plastics composition substantially forming a basic shape of the hip prosthesis (1) is fed into a negative mould of the hip prosthesis at an inlet end and the excess amount of the plastics composition is discharged at an outlet end of the negative mould of the hip prosthesis.

19. Process for the production of an endoprosthesis stem, in accordance with any one of claims 1 to 17,
**characterized in that**
moulding of the main body is carried out in a push-pull injection-moulding cycle, wherein a plastics composition substantially forming a basic shape of the hip prosthesis (1) is fed alternately in push-pull operation from two different ends of a negative mould.

20. Process according to claim 18,
**characterized in that**
the plastics composition is fed in continuously at an inlet end of the negative mould.

21. Process according to claim 18 or 20,
**characterized in that**
the plastics composition is fed in at a constant speed during a shape-forming operation.

22. Process according to claim 18 or 20,
**characterized in that**
the volumetric flow of the plastics composition flowing through the negative mould is kept constant during the shape-forming operation.

23. Process according to any one of claims 18 or 20 to 22,
**characterized in that**
solidification of the plastics composition in the negative mould takes place from the outside to the inside, the plastics composition being fed in until the negative mould has been filled to a predetermined extent.

24. Process according to any one of claims 18 or 20 to 23,
**characterized in that**
the negative mould is filled completely with the plastics composition.

25. Process according to any one of claims 18 or 20 to 24,
**characterized in that**
the speed and/or amount of the plastics composition flowing out of the outlet end of the negative mould is regulated using at least one valve.

26. Process according to any one of claims 18 or 20 to 25,
**characterized in that**
the plastics composition is introduced into the negative mould of the hip prosthesis at a temperature in the range of from 350°C to 450°C, preferably in the range of from 400°C to 335°C, especially at a temperature in the range of from 415°C to 430°C.

27. Process according to any one of claims 18 or 20 to 26,
**characterized in that**
the negative mould is maintained at a temperature in the range of from 120°C to 250°C, preferably in the range of from 170°C to 230°C, especially in the range of from 190°C to 210°C, during the filling operation.

28. Process according to any one of claims 18 to 27,
**characterized in that**
the covering layer (2) is fused into the surface of the carbon-fibre-reinforced plastics material.

29. Process according to any one of claims 18 to 28,
**characterized in that**
the covering layer (3) is blasted and/or pressed and/or sprayed onto the surface layer of the carbon-fibre-reinforced plastics material in the form of metal particles.

## Revendications

1. Tige endoprothétique, en particulier tige d'une prothèse de la hanche (1) avec un corps de base en matière plastique renforcée par des fibres, **caractérisée en ce que** le corps de base est réalisé par un procédé de moulage par injection sous la forme d'un corps isoélastique, qui est adapté aux conditions de forces physiologiques dans le fémur, et auquel est conférée une résistance prédéterminée due pour l'essentiel uniquement à une épaisse couche superficielle contenant des fibres de renfort, en particulier des fibres de carbone, uniformément orientées dans le sens longitudinal du corps de base, la dimension radiale de cette couche étant, sur la majeure partie de la longueur du corps de base, supérieure à la dimension radiale d'une zone centrale contenant des fibres de renfort non uniformément orientées.

2. Tige endoprothétique selon la revendication 1, **caractérisée par** un mince revêtement métallique et/ou céramique sensiblement sur toute la surface du corps de base, destiné à entrer en contact avec le fémur, en vue de garantir la biocompatibilité.

3. Tige endoprothétique selon la revendication 2, **caractérisée en ce que** le revêtement métallique comporte une couche enveloppante (2) intérieure et une couche structurée (3) extérieure, la couche enveloppante (2) et/ou la couche structurée (3) contenant un métal, en particulier le titane ou un alliage de titane, inerte et/ou biocompatible dans les conditions physiologiques.

4. Tige endoprothétique selon la revendication 2 ou 3, **caractérisée en ce que** le revêtement céramique (4) comporte un matériau à base de phosphate de calcium, en particulier hydroxylapatite.

5. Tige endoprothétique selon l'une quelconque des revendications 2 à 4, **caractérisée en ce qu'**un revêtement céramique (4) est déposé sur au moins un revêtement métallique.

6. Tige endoprothétique selon l'une quelconque des revendications 2 à 5, **caractérisée en ce que** la couche enveloppante (2) est réalisée avec une première rugosité prédéterminée et la couche structurée (3) est réalisée avec une deuxième rugosité prédéterminée.

7. Tige endoprothétique selon l'une quelconque des revendications 3 à 6, **caractérisée en ce que** la couche enveloppante (2) est formée de manière intimement liée à une couche superficielle réalisée en matière plastique renforcée par des fibres, en particulier en particules de métal scellées dans la surface de la matière plastique renforcée par des fibres.

8. Tige endoprothétique selon l'une quelconque des revendications 3 à 7, **caractérisée en ce que** la couche enveloppante (2) a une épaisseur de couche dans une plage de 40 µm à 120 µm, de préférence dans une plage de 60 µm à 100 µm, et de manière particulièrement préférée dans une plage de 75 µm à 85 µm.

9. Tige endoprothétique selon l'une quelconque des revendications 3 à 8, **caractérisée en ce que** la couche enveloppante (2) a une profondeur de rugosité dans une plage de 20 µm à 70 µm.

10. Tige endoprothétique selon l'une quelconque des revendications 3 à 9, **caractérisée en ce que** la couche structurée (3) a une épaisseur de couche dans une plage de 150 µm à 250 µm, de préférence dans une plage de 170 µm à 230 µm, et de manière particulièrement préférée dans une plage de 190 µm à 210 µm.

11. Tige endoprothétique selon l'une quelconque des revendications 3 à 10, **caractérisée en ce que** la couche structurée (3) a une profondeur de rugosité dans une plage de 50 µm à 180 µm.

12. Tige endoprothétique selon l'une quelconque des revendications 2 à 11, **caractérisée en ce que** la couche (4) réalisée dans le matériau à base de phosphate de calcium a une épaisseur de couche dans une plage de 40 µm à 100 µm, de préférence dans une plage de 50 µm à 80 µm, et de manière particulièrement préférée dans une plage de 65 µm à 75 µm.

13. Tige endoprothétique selon l'une quelconque des revendications 3 à 12, **caractérisée en ce que** la couche enveloppante (2) est prévue pour l'essentiel uniquement dans une zone proximale et dans une zone intermédiaire située entre une zone distale et la zone proximale.

14. Tige endoprothétique selon l'une quelconque des revendications 3 à 13, **caractérisée en ce que** la couche structurée (3) est prévue pour l'essentiel uniquement dans la zone intermédiaire située entre la zone distale et la zone proximale.

15. Tige endoprothétique selon l'une quelconque des revendications 2 à 14, **caractérisée en ce que** le revêtement céramique (4) est prévu pour l'essentiel uniquement dans la zone intermédiaire située entre la zone distale et la zone proximale.

16. Tige endoprothétique selon l'une quelconque des revendications précédentes, **caractérisée en ce que** la proportion de fibres de renfort, en particulier la proportion de fibres de carbone dans la matière plastique, se situe dans une plage de 20 % en poids à 80 % en poids, de préférence dans une plage de 30 % en poids à 70 % en poids, et de manière particulièrement préférée dans une plage de 40 % en poids à 55 % en poids.

17. Tige endoprothétique selon l'une quelconque des revendications précédentes, **caractérisée en ce que** la longueur des fibres de renfort se situe dans une plage de 10 µm à 800 µm, de préférence dans une plage de 40 µm à 700 µm, et de manière particulièrement préférée dans une plage de 50 µm à 560 µm.

18. Procédé de fabrication d'une tige endoprothétique selon l'une quelconque des revendications précédentes, **caractérisé en ce qu'**un moulage de la prothèse de la hanche (1) est mis en oeuvre dans un cycle de moulage par injection en continu, dans lequel une masse de matière plastique, constituant sensiblement une forme de base de la prothèse de la hanche (1), est acheminée au niveau d'une extrémité d'entrée dans une empreinte négative de la prothèse de la hanche et une part excédentaire de la masse de matière plastique est évacuée au niveau d'une extrémité de sortie de l'empreinte négative de la prothèse de la hanche.

19. Procédé de fabrication d'une tige endoprothétique selon l'une quelconque des revendications 1 à 17, **caractérisé en ce qu'**un moulage de la prothèse de la hanche (1) est mis en oeuvre dans un cycle de moulage par injection à cadence alternée, dans lequel une masse de matière plastique, constituant sensiblement une forme de base de la prothèse de la hanche (1), est acheminée en alternance depuis deux extrémités différentes d'une empreinte négative.

20. Procédé selon la revendication 18, **caractérisé en ce que** la masse de matière plastique est acheminée en continu par une extrémité d'entrée de l'empreinte négative.

21. Procédé selon la revendication 18 ou 20, **caractérisé en ce que** la masse de matière plastique est acheminée avec une vitesse constante pendant un processus de moulage.

22. Procédé selon la revendication 18 ou 20, **caractérisé en ce que** la masse de matière plastique afflue à travers l'empreinte négative avec un flux volumétrique constant pendant le processus de moulage.

23. Procédé selon la revendication 18 ou 20 à 22, **caractérisé en ce qu'**une consolidation de la masse de matière plastique dans l'empreinte négative est effectuée de l'extérieur vers l'intérieur, la masse de matière plastique étant acheminée jusqu'à ce qu'un remplissage prédéterminé de l'empreinte négative soit obtenu.

24. Procédé selon la revendication 18 ou 20 à 23, **caractérisé en ce que** l'empreinte négative est entièrement remplie de matière plastique.

25. Procédé selon la revendication 18 ou 20 à 24, **caractérisé en ce que** la vitesse d'évacuation et/ou la quantité de masse de matière plastique évacuée hors de l'extrémité de sortie de l'empreinte négative sont réglées par au moins une vanne.

26. Procédé selon la revendication 18 ou 20 à 25, **caractérisé en ce que** la masse de matière plastique est introduite dans l'empreinte négative de la prothèse de la hanche avec une température dans une plage de 350°C à 450°C, de préférence dans une plage de 400°C à 335°C, et de manière particulièrement préférée dans une plage de 415°C à 430°C.

27. Procédé selon la revendication 18 ou 20 à 26, **caractérisé en ce que**, pendant le processus de remplissage, l'empreinte négative est maintenue à une température dans une plage de 120°C à 250°C, de préférence dans une plage de 170°C à 230°C, et de manière particulièrement préférée dans une plage de 190°C à 210°C.

28. Procédé selon l'une quelconque des revendications 18 à 27, **caractérisé en ce que** la couche enveloppante (2) est scellée dans la surface de la matière plastique renforcée par des fibres de carbone.

29. Procédé selon l'une quelconque des revendications 18 à 28, **caractérisé en ce que** la couche structurée (3) est pulvérisée, et/ou pressée, et/ou projetée sous forme de particules de métal sur la couche superficielle réalisée en matière plastique renforcée par des fibres de carbone.
